(19) Europäisches Patentamt
European Patent Office
Office européen des brevets

(11) **EP 3 875 155 A1**

(12) **EUROPEAN PATENT APPLICATION**
published in accordance with Art. 153(4) EPC

(43) Date of publication:
**08.09.2021 Bulletin 2021/36**

(21) Application number: **19880455.1**

(22) Date of filing: **31.10.2019**

(51) Int Cl.:
*A61Q 5/00* (2006.01)   *A61Q 5/06* (2006.01)
*A61Q 5/12* (2006.01)   *A61K 8/34* (2006.01)
*A61K 8/40* (2006.01)   *A61K 8/894* (2006.01)

(86) International application number:
**PCT/JP2019/042714**

(87) International publication number:
**WO 2020/090951 (07.05.2020 Gazette 2020/19)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **02.11.2018 JP 2018206972**
**02.11.2018 JP 2018206973**
**13.12.2018 JP 2018233519**

(71) Applicant: **Shiseido Company, Ltd.**
**Chuo-ku**
**Tokyo 104-0061 (JP)**

(72) Inventors:
• **KURASHIMA, Takumi**
  **Tokyo 104-0061 (JP)**
• **KOJIRI, Momoka**
  **Tokyo 104-0061 (JP)**
• **YOSHIBA, Ryo**
  **Tokyo 104-0061 (JP)**

(74) Representative: **Ter Meer Steinmeister & Partner**
**Patentanwälte mbB**
**Nymphenburger Straße 4**
**80335 München (DE)**

(54) **HAIR TREATMENT METHOD**

(57)    The purpose of the present invention is to provide a hair treatment method that permits a suitable amount of a component having a hair treatment effect to be deposited uniformly on the hair, and that has exceptional persistence of effects in which hydrophobicity, combability, and smoothness is restored by appropriate heat treatment to hair that has been damaged and in which these effects can be maintained for an extended period of time. The present invention pertains to a hair treatment method characterized by including: (1) a step for applying, to hair, a hair treatment agent containing a copolymer that includes a polysiloxane structure and a polyoxyalkylene structure in the main chain, the copolymer having a side chain that has a trialkoxysilane group or a silanol group; (2) a step for subjecting the hair to heat treatment at 40-230°C; and (3) a step for washing the hair.

EP 3 875 155 A1

**Description**

Technical Field

The present invention relates to a hair treatment method. More specifically, the present invention relates to a hair treatment method capable of imparting smoothness, easiness in combing, water repellency and the like to hair. The method of the present invention also capable of lasting these effects for a long time.

Background Art

Various hair care components such as silicones are mixed in hair washing cosmetics such as shampoos and conditioners, and hair finishing cosmetics such as hair styling agents and hair treatments. These hair care components adhere to damaged parts of hair, repair and coat the hair, and thereby allows improved easiness in combing.
For a hair treatment method carried out for the purpose of the hair treatments as described above, long-term lasting (holding) of the effects is required particularly when the hair treatment method is carried out in hair salons and the like.
However, polymers mixed in the conventional hair cosmetics as hair care components were capable of repairing easiness in combing hair immediately after used, but most of them were insufficient in an aspect of lasting effects for an extended period of time and an aspect of a feel of use because they were easily washed away by washing hair.
Patent Document 1 discloses a hair cosmetic in which a vinyl monomer having a reactive alkoxysilane group, a lipophilic vinyl monomer having an alkyl group and the like and a cationic polymer emulsion obtained by emulsion polymerizing an acrylic hydrophilic monomer using a cationic emulsifier are mixed. This cationic polymer is considered to form a solid coat when the alkoxysilane group is converted to a silanol group through hydrolysis and form a crosslinked structure via a dehydration condensation reaction. However, the backbone of this polymer was a vinyl polymer having a polyvinyl structure, and thus a resin feel was obvious, hair was stiff and had a coarse feel, hence a sense of use was insufficient.
Patent Document 2 describes a hair treatment method which uses a composition in which a silicone polymer having an alkoxy-(aminomethyl)-silyl group at terminal is mixed. The silicone polymer used here is considered to hold a hair form for a long time and demonstrates good aesthetic characteristics. When a treatment is performed using a treatment agent in which an alkoxysilane component is mixed, uneven attachment and overattachment to hair sometimes deteriorated a sense of use.

Citation List

Patent Document

    Patent Document 1: JP-B 4805482

    Patent Document 2: JP-A 2016-525534

Summary of Invention

Technical Problem

Thus, an object of the present invention is to provide a hair treatment method capable of uniformly attaching an appropriate amount of components having hair treatment effects on hair, repairing hydrophobicity, easiness in combing and smoothness of damaged hair by suitably carrying out heat treatment and also having good lasting effects (effect-holding properties) for retaining the effects for an extended period of time.

Solution to Problem

The present inventors have found that a hair treatment, which has good lasting effects and demonstrates a high water repellent effect and a smooth sense on use, is enabled by using a hair treatment agent containing a predetermined amount of a copolymer including a main chain having a polysiloxane structure and a polyoxyalkylene structure and a side chain having a trialkoxysilane group or a silanol group and carrying out a rinsing off step and a heating step in a timely manner, and whereby the present invention was accomplished.

That is, the present invention provides a hair treatment method comprising:

(1) a step of applying a hair treatment agent to hair, the hair treatment agent comprising a copolymer including a main chain having a polysiloxane structure and a polyoxyalkylene structure and a side chain having a trialkoxysilane group or a silanol group;
(2) a step of performing heat treatment to the hair at 40°C to 230°C; and
(3) a step of rinsing off the hair.

Advantageous Effect of Invention

The hair treatment method of the present invention prevents hair treatment components from excessively remaining on the hair surface by using a hair treatment agent containing a predetermined concentration of a copolymer having a specific structure and providing a step of rinsing off before or after heating. Further, the effects imparted by the hair treatment of the present invention are retained for a long time. The hair treatment method of the present invention can immediately repair hair damaged by a chemical treatment and recover smoothness and the like by performing the chemical treatment step such as permanent wave forming treatment, curly hair correction, dyeing or bleaching before the step of applying the hair treatment agent.

Further, the copolymer having a specific structure used in the present invention imparts to the hair surface hydrophobicity (water repellency), fast-drying and a silky feel, easiness in arrangement and improves heat resistance, chemical resistance and contamination resistance (pollen and the like), and these effects can be held for an extended period of time.

Description of Embodiments

The hair treatment method of the present invention comprises (1) a step of applying a hair treatment agent, (2) a heat treatment step, and (3) a step of rinsing off. Hereinafter, each step is described in detail.

(1) Step of applying a hair treatment agent

In the hair treatment method according to the present invention, a hair treatment agent is first applied to hair.
The hair treatment agent used in the present invention contains a copolymer including a main chain (backbone) having a polysiloxane structure and a polyoxyalkylene structure and a side chain having a trialkoxysilane group or a silanol group (hereinafter abbreviated as "crosslinked silicone POA copolymer").
The crosslinked silicone POA copolymer has good adhesiveness to hair due to the hydrophilicity of the polyoxyalkylene structure present in the main chain and imparts hydrophobicity (water repellency) to the hair surface due to the polysiloxane structure present in the main chain, and has good lasting effects because the alkoxysilane group or the silanol group present in the side chain crosslinks at a comparatively low temperature thereby forming a solid coat.
The polysiloxane structure constituting the main chain (backbone) of the crosslinked silicone POA copolymer comprises polydialkylsiloxane, preferably polydimethylsiloxane, and a part of the alkyl group (preferably the methyl group) may be substituted with a phenyl group.
The polyoxyalkylene structure constituting the main chain (backbone) of the crosslinked silicone POA copolymer of the present invention is preferably a structure including, as a repeating unit, at least one selected from the group consisting of an oxyethylene group (EO), an oxypropylene group (PO), and an oxybutylene group (BO).
The crosslinked silicone POA copolymer in the present invention preferably further has a side chain comprising an organic group. Examples of the organic group to be the side chain include a hydrocarbon group (preferably an alkyl group such as a straight chain or branched chain alkyl group having about 1 to 30 carbon atoms, or a phenyl group) which can be optionally substituted with an amino group, a hydroxyl group, a carboxyl group, and the like. The hydrocarbon group preferably has an amino group, and a hydrogen atom of such an amino group can further be replaced with an alkyl group and the like.
The crosslinked silicone POA copolymer of the present invention preferably further includes a nitrogen atom in the main chain (backbone), and the side chain preferably bonds to such a nitrogen atom.
Further specific examples of the crosslinked silicone POA copolymer include polysilicone-29 (INCI name). Polysilicone-29 is defined as a complex silicone compound obtained by the reaction between a glycidopropyl-terminated dimethyl siloxane polymer and PEG-13 diglycidyl ether, diethylaminopropylamine, and aminopropyltriisopropoxysilane.
A commercial product can be used as the crosslinked silicone POA copolymer in the present invention. For example, a copolymer contained in a hair conditioning agent under the product name "Silsoft CLX-E" sold by Momentive Performance Materials Inc. can be particularly preferably used. This copolymer is a compound belonging to "Polysilicone-29." Such a copolymer has a structure in which the main chain (backbone) includes a polysiloxane structure, a polyoxyalkylene structure and a nitrogen atom, a side chain has a trialkoxysilane group and a side chain comprises an organic group, wherein the polysiloxane structure includes polydimethylsiloxane, the polyoxyalkylene structure includes polyoxyethylene and polyoxyisopropylene, and the side chains bond to the nitrogen atom of the main chain (backbone). "Silsoft CLX-E" is a product containing the copolymer, dipropylene glycol and water.

A mixing amount of the crosslinked silicone POA copolymer in the hair treatment agent used in the present invention based on the hair treatment agent is a concentration of 0.05 mass% or more, and preferably 0.1 mass% or more. For example, the mixing amount can also be 0.5 mass% or more, or 1 mass% or more. The copolymer can fully coat throughout hair due to the affinity for the hair of such a copolymer and the step of rinsing off to be described later, when carried out, removes an excess of the copolymer thereby to prevent the hair from being coarse. The upper limit value of the mixing amount of the crosslinked silicone POA copolymer is typically 5.0 mass% or less, preferably 3.0 mass% or less, and more preferably 2.0 mass% or less. When a mixing amount is less than 0.05 mass%, intended effects cannot be obtained, whereas when a mixing amount is more than 5.0 mass%, a sense of use may be reduced.

The hair treatment agent in the present invention can be prepared in the form of creams, mists, mousses, gels, sprays or the like, and is not limited thereto. Other components in accordance with each form may optionally be mixed in the hair treatment agent. The hair treatment agent is preferably provided in the form of creams, mists, or gels.

For example, for the formation of a cream, it is preferable that the crosslinked silicone POA copolymer, water, a higher alcohol, and a surfactant be mixed. A cationic surfactant such as quaternary ammonium salts is preferably included as the surfactant. The mixing amount range of the cationic surfactant based on the total amount of the hair treatment agent is preferably 0.1 to 2.0 mass%, and more preferably 0.2 to 1.5 mass%. Further, in addition to the cationic surfactant, a nonionic surfactant and/or an amphoteric surfactant can be included. A mixing amount of a nonionic surfactant or an amphoteric surfactant is not particularly limited but, based on the total amount of the hair treatment agent, is typically 0.1 to 3.0 mass%, and more preferably 0.3 to 1.5 mass%. On the other hand, when an anionic surfactant is used alone as the surfactant, desired effects may not be obtained, for this reason it is preferable to avoid mixing an anionic surfactant singly. A mixing amount of the higher alcohol based on the total amount of the hair treatment agent is typically 0.1 to 20 mass%, and preferably 1.0 to 10 mass%. Water, not particularly limited though, is preferably mixed in 5 mass% or more based on the total amount of the hair treatment agent, and typically about 50 to 90 mass%.

For the formation of a mist, the crosslinked silicone POA copolymer, a lower alcohol, and a surfactant are preferably mixed. The surfactant for a mist is preferably a cationic surfactant such as quaternary ammonium salts. The mixing amount of a cationic surfactant based on the total amount of the hair treatment agent is preferably 0.01 to 1.0 mass%, more preferably 0.03 to 0.5 mass%, and further preferably 0.05 to 0.5 mass%, from a viewpoint of the safety and a feel of use. The lower alcohol means the alcohols having 5 or less carbon atoms such as ethanol, and a mixing amount of the lower alcohol based on the total amount of the hair treatment agent is, from a viewpoint of the safety and effects, preferably 10 to 60 mass%, and more preferably 20 to 50 mass%.

Other optional components mixed in the hair treatment agent of the present invention can be suitably selected from components typically used for cosmetic products and pharmaceutical products. However, the mixing amount thereof should be within a range which does not affect the effects of the present invention. Examples of such optional components are not limited and include oily components, powder components, natural polymers, synthetic polymers, thickeners, UV absorbers, moisturizers, sequestering agents, pH adjusting agents, skin nutrients, vitamins, antioxidants, antioxidant aids, and perfumes.

In this step, the hair treatment agent as described above is applied to hair by a method suitable for the form thereof. When the hair treatment agent is a cream, a mousse or a gel, the hair treatment agent is taken in one's hand in any manner, applied to hair and spread out throughout the hair. When the hair treatment agent is a mist or a spray, a container suitable for each form is filled with the agent, then the agent is sprayed to hair, and spread out throughout the hair with hands in any manner.

(2) Heat treatment step

In the heat treatment step, the hair to which the hair treatment agent is applied is heated to a predetermined temperature.

The heat treatment temperature is 40°C to 230°C, preferably 60°C to 200°C, more preferably 60°C to 180°C, further preferably 80°C to 150°C, and most preferably 80°C to 120°C. At less than 40°C, the lasting effects become insufficient. At more than 230°C, hair may be damaged by heat likely causing a notable color change (color fading) particularly to the dyed (colored) hair.

The heating device (or heating means) is not particularly limited but a hair iron (flat iron) or a blow dryer is preferably used.

(3) Step of rinsing off

In (3) the step of rinsing off in the hair treatment method of the present invention, the hair to which the hair treatment agent has been applied is rinsed off. Water is preferably used for the rinsing off but the rinsing off can be carried out with water containing a cleansing component such as a surfactant.

The rinsing off can remove the hair treatment agent overapplied to the hair surface and provide a good finishing.

In the hair treatment method of the present invention, the hair to which the hair treatment agent has been applied in the step (1) can be subjected to (3) the step of rinsing off after subjected to (2) the heat treatment step or can be subjected to (2) the heat treatment step after subjected to (3) the step of rinsing off. However, when either one of (2) the heat treatment step or (3) the step of rinsing off lacks, the hair is sticky after the treatment thereby posing a problem of a sense of use. In particular, the lasting effects notably reduce when the heat treatment is not performed.

Further, hair is preferably dried before the heating step.

(4) Chemical treatment step

In the hair treatment method of the present invention, a chemical treatment step such as hair dyeing (coloring), permanent wave forming treatment, or curly hair correction (hair straightening) can be inserted. The chemical treatment preferably includes at least one selected from the following hair dyeing (coloring) treatment, permanent wave forming treatment, and curly hair correction (hair straightening) treatment and, for example, the hair dye treatment and the permanent wave forming treatment or the curly hair correction treatment can be carried out in combination.

• Hair dyeing (Coloring)

The hair dye (coloring) treatment in the present invention is defined to include a permanent hair dye treatment represented by oxidation dyeing, which uses a first solution containing an oxidation dye and a second solution containing an oxidizing agent such as hydrogen peroxide, and a hair bleaching (color removal) treatment, which uses a first solution containing an alkaline agent and a second solution containing hydrogen peroxide. Further, a temporary hair dye treatment, which uses a coloring agent and an acid dye such as pigments, can be acceptable.

• Permanent wave forming treatment

The permanent wave forming treatment in the present invention includes a treatment for forming permanent waves which uses a first solution containing a reducing agent represented by thioglycolic acid and cysteine and a second solution containing an oxidizing agent. Further, a straightening perm treatment, which uses the reducing agent and the oxidizing agent, is acceptable.

• Curly hair correction (hair straightening)

The curly hair correction (hair straightening) treatment in the present invention means, for example, a treatment which uses a treatment agent having glyoxylic acid as the primary component and does not include the straightening perm treatment which uses the above reducing agent and the oxidizing agent. The curly hair correction treatment agent which uses glyoxylic acid can be those with an improved correction effect by further mixing guanidine salt and/or urea (see Japanese Patent No. 5947340).

In the hair treatment method of the present invention, the chemical treatment step as described above is preferably carried out before the step (1) of applying the hair treatment agent. When (4) the chemical treatment is carried out after (1) the application of the hair treatment agent, (2) the heat treatment and (3) the rinsing off, the penetration of a chemical treatment agent into hair is reduced thereby likely failing to obtain sufficient effects.

On the other hand, when the hair treatment method of the present invention (steps (1) to (3)) is carried out in continuation of the chemical treatment step, hair damaged by chemical treatment agents can be well repaired. The step (1) of the hair treatment method of the present invention can be carried out immediately after (4) the chemical treatment step is completed (for example, within 30 minutes, within 10 minutes or within 5 minutes), and a rinsing step can be optionally interposed therebetween.

When the hair treatment method of the present invention (steps (1) to (3)) is carried out after the hair dyeing (coloring) treatment, the heating temperature in (2) the heat treatment step is preferably low (for examples, 180°C or less, preferably 150°C or less, and more preferably 120°C or less) from a viewpoint of reducing color fading.

Examples

Hereinafter, the present invention will be further described in detail in reference to Examples but the present invention is not limited to these Examples. Note that the mixing amount is shown in mass% unless otherwise specified.

The hair treatment agent of each Example was prepared in accordance with a routine method with the formulations shown in Table 1 and Table 2 below.

[Table 1]

|  | Hair treatment agent 1 |
|---|---|
| Crosslinked silicone POA copolymer (*1) | 1.5 |
| Ethanol | 20 |
| Stearyltrimethylammonium chloride | 0.2 |
| PEG-60 Hydrogenated castor oil | 0.3 |
| Phenoxy ethanol | 0.2 |
| Perfume | 0.1 |
| Dipropylene glycol | 5 |
| Lactic acid | 0.05 |
| Sodium lactate | 0.05 |
| Water | 72.6 |
| Total | 100 |
| (*1) Polysilicone-29 | |

[Table 2]

|  | Hair treatment agent 2 |
|---|---|
| Crosslinked silicone POA copolymer (*1) | 1 |
| Behenyltrimethylammonium chloride | 0.4 |
| Stearyl alcohol | 1 |
| Behenyl alcohol | 3 |
| Isopropyl myristate | 0.2 |
| Phenoxy ethanol | 0.5 |
| Perfume | 0.2 |
| Glycerol | 5 |
| Dipropylene glycol | 6 |
| Citric acid | 0.1 |
| Sodium citrate | 0.1 |
| Water | 82.5 |
| Total | 100 |
| (*1) Polysilicone-29 | |

Subsequently, each of the treatment steps was carried out on a hair sample by the following method. The application order of each step is as shown in Table 3 and Table 4.

The hair sample used for the evaluation is as follows.

- Dyeing (coloring treatment): human gray hair strand having a length of 8 cm and a weight of 1 g
- Permanent wave forming treatment: human hair strand having a length of 30 cm and a weight of 2 g
- No dyeing or permanent wave treatment is carried out: damaged hair strand having a length of 15 cm and a weight of 0.5 g

Details of each step are as follows.

(1) Step of applying the hair treatment agent (application step)
The hair treatment agent is applied to the hair sample.
(2) Heat treatment step (heating step)
The hair sample is heated at a predetermined temperature. The heating temperature and the heating device are shown in Table 3 and Table 4.
(3) Step of rinsing off (rinsing with water step)
The hair sample is rinsed off with water.
(4) Chemical treatment step

- Dyeing (coloring) treatment: hair dye is carried out using an acid hair dye (Hair PRIMIENCE, manufactured by Shiseido Professional).
- Permanent wave forming treatment: permanent waves are formed using a perm solution (CREATOR, manufactured by Shiseido Professional).
- Curly hair correction (hair straightening) treatment: curly hair correction treatment is carried out using a glyoxylic acid-containing treatment solution.

[Table 3]

| Performance order | Example 1 | Example 2 | Comparative Example 1 | Example 3 | Example 4 | Example 5 |
|---|---|---|---|---|---|---|
| First step | Chemical treatment | Chemical treatment | Chemical treatment | Chemical treatment | Chemical treatment | Chemical treatment |
| Second step | Application | Application | Application | Application | Application | Application |
| Third step | Drying | Rinsing with water | Drying | Drying | Drying | Drying |
| Fourth step | Heating 180°C Hair Iron | Drying | Heating 240°C Hair Iron | Heating 200°C Hair Iron | Heating 150°C Hair Iron | Heating 120°C Hair Iron |
| Fifth step | Rinsing with water | Heating 180°C Hair Iron | Rinsing with water | Rinsing with water | Rinsing with water | Rinsing with water |
| Evaluation result | | | | | | |
| Sense of use | A | B | A | A | A | A |
| Lasting effect | A | B | A | A | A | A |
| Color impact | B | B | D | C | B | A |
| Perm impact | B | B | B | B | A | A |

[Table 4]

| Performance order | Example 6 | Comparative Example2 | Comparative Example3 | Comparative Example4 | Comparative Example5 |
|---|---|---|---|---|---|
| First step | Chemical treatment | Chemical treatment | Chemical treatment | Application | Chemical treatment |

(continued)

| Performance order | Example 6 | Comparative Example2 | Comparative Example3 | Comparative Example4 | Comparative Example5 |
|---|---|---|---|---|---|
| Second step | Application | Application | Application | Drying | 80°C Blow dryer |
| Third step | Drying | Drying Room temperature (25°C) | Drying | Heating 180°C Hair Iron | - |
| Fourth step | Heating 80°C Blow dryer | Rinsing with water | Heating 180°C Hair Iron | Rinsing with water | - |
| Fifth step | Rinsing with water | - | - | Chemical treatment | - |
| Evaluation result | | | | | |
| Sense of use | A | C | D | B | - |
| Lasting effect | B | D | A | B | - |
| Color impact | A | A | B | B | - |
| Perm impact | A | A | B | D | - |

The hair samples subjected to each of the steps in the order shown in above Table 3 and Table 4 were evaluated from the following viewpoints. The evaluation results are shown in Table 3 and Table 4. Evaluation C indicates no practical problem is caused, Evaluation B indicates good, and Evaluation A indicates much better (excellent). On the other hand, Evaluation D indicates the level at which practical problems may be caused when produced as a product.

<Sense of use>

Trained researchers touched and felt hair samples and evaluated by the following evaluation criteria.
Evaluation criteria:

A: Hair had no coarseness or stickiness and had very smooth feeling when touched with hands.

B: Hair had no coarseness or stickiness and had smooth feeling when touched with hands.

C: Hair had rather coarseness and stickiness but had smooth feeling when touched with hands.

D: Hair had coarseness and stickiness and did not have smooth feeling when touched with hands.

<lasting effects>

Two $\mu$l of water was added dropwise to the surface of the hair sample, and the water drops were photographed under a microscope. From the photographed images, left and right angles of water drops on hair were measured, and an average value thereof was defined as a single measurement value. The same measurement was repeated 3 times, and an average value thereof was defined as a value of contact angle.
Subsequently, shampoo was repeated using a 10% SLES solution shampoo, which was free of a conditioning component, and then the same measurement as above was carried out to calculate a contact angle change rate (following formula).

$$\text{Contact angle change rate (\%)} = (\text{contact angle after washed/contact angle before washed}) \times 100$$

Lasting effects were evaluated as follows based on the contact angle change rates calculated by the above formula.

A: Contact angle change rate of 90% or more.
B: Contact angle change rate of 80% or more and less than 90%.
C: Contact angle change rate of 70% or more and less than 80%.
D: Contact angle change rate of less than 70%.

<Impacts on chemical treatments>

• Impact on dyeing (coloring) <Color impact>

Color tone of the hair sample treated in each Example was visually compared based on Comparative Example 5 (Table 4) in which only the dyeing was carried out as the chemical treatment, and the color tone changes were evaluated by the following criteria.

A: No color change at all in comparison with Comparative Example 5.
B: Substantially no color change in comparison with Comparative Example 5.
C: Slight color change recognized in comparison with Comparative Example 5.
D: Significant color change identified in comparison with Comparative Example 5.

• Impact on permanent wave forming treatment <Perm impact>

Degree of the permanent waves formed of the hair sample treated in each Example was visually compared based on Comparative Example 5 (Table 4) in which only the permanent wave forming treatment was carried out as the chemical treatment, and the changes were evaluated by the following criteria.

A: No change at all in the degree of permanent waves formed in comparison with Comparative Example 5.
B: Substantially no change in the degree of permanent waves formed in comparison with Comparative Example 5.
C: Slightly poor degree of permanent waves formed in comparison with Comparative Example 5.
D: Notably poor degree of permanent waves formed in comparison with Comparative Example 5.

As shown in Table 3 and Table 4, the hair samples subjected to (1) the application of the hair treatment agent followed by (2) heating and (3) rinsing with water had the hydrophobized surface and a good sense of use with smooth feeling when touched with hands. Further, it was also confirmed that the lasting effects thereof were good. It was revealed that the effects can be obtained even when the rinsing with water is carried out after the heating or the heating is performed after the rinsing with water (Examples 1 and 2).

On the other hand, Comparative Example 2 in which the heat treatment was not performed had slight stickiness and coarseness on hair and insufficient lasting effects. Comparative Example 3 in which the rinsing with water was not carried out failed to impart smoothness to hair and a sticky feel remained.

Comparative Example 4 in which the chemical treatments were carried out after the treatment method of the present invention caused detrimental impacts particularly on the permanent wave forming treatment. Further, when the dyeing (coloring) as the chemical treatment was carried out, notable color fading (color tone change) was caused at a temperature of more than 230°C (Comparative Example 1) in the heat treatment step, whereas color fading was reduced to an allowable range at a heating temperature of 200°C (Example 3) and color fading hardly occurred at 180°C or less (Examples 1 and 4 to 6).

Further, the results equivalent to the above were obtained even when the hair treatment agent 2 with the formulation shown in Table 2 was used as the hair treatment agent. The same applied even when the curly hair correction treatment using glyoxylic acid was carried out as the chemical treatment in continuation of the hair dyeing (coloring) or the permanent wave forming.

## Claims

1.   A hair treatment method comprising:

(1) a step of applying a hair treatment agent to hair, the hair treatment agent comprising a copolymer including a main chain having a polysiloxane structure and a polyoxyalkylene structure and a side chain having a trialkoxysilane group or a silanol group;
(2) a step of performing heat treatment to the hair at 40°C to 230°C; and
(3) a step of rinsing off the hair.

2. The hair treatment method according to claim 1, wherein the step (1) is carried out, then the step (2) is carried out, and then the step (3) is carried out.

3. The hair treatment method according to claim 1, wherein the step (1) is carried out, then the step (3) is carried out, and then the step (2) is carried out.

4. The hair treatment method according to any one of claims 1 to 3, comprising a step of drying the hair before the step (2).

5. The hair treatment method according to any one of claims 1 to 4, comprising at least one chemical treatment step selected from the group consisting of hair dyeing treatment, permanent wave forming treatment, and curly hair correction treatment before the step (1).

6. The hair treatment method according to claim 5, wherein the chemical treatment step includes hair dyeing treatment and a heating temperature in (2) the heat treatment step is 180°C or less.

## INTERNATIONAL SEARCH REPORT

| International application No. |
| --- |
| PCT/JP2019/042714 |

**A. CLASSIFICATION OF SUBJECT MATTER**
A61Q 5/00(2006.01)i; A61Q 5/06(2006.01)i; A61Q 5/12(2006.01)i; A61K 8/34(2006.01)i; A61K 8/40(2006.01)i; A61K 8/894(2006.01)i
FI: A61K8/894; A61K8/34; A61K8/40; A61Q5/00; A61Q5/12; A61Q5/06
According to International Patent Classification (IPC) or to both national classification and IPC

**B. FIELDS SEARCHED**

Minimum documentation searched (classification system followed by classification symbols)
A61Q5/00; A61Q5/06; A61Q5/12; A61K8/34; A61K8/40; A61K8/894

| Documentation searched other than minimum documentation to the extent that such documents are included in the fields searched | |
| --- | --- |
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
Mintel GNPD

**C. DOCUMENTS CONSIDERED TO BE RELEVANT**

| Category* | Citation of document, with indication, where appropriate, of the relevant passages | Relevant to claim No. |
| --- | --- | --- |
| X | Marketing Bulletin SILSOFT CLX-E conditioning agent SPECIALTY FLUIDS-PERSONAL CARE, MOMENTIVE PERFORMANCE MATERIALS INC., 2015, pp. 1-12 page 1 "Key Features and Typical Benefits", "Potential Applications", page 2 "Chemistry", page 6 "3. Progressive Thermal Relaxing", page 7 "4. Thermal Protection" | 1-2, 4-6 |
| X | Salt-Free Shampoo(ID:5387529), Mintel GNPD [online], January 2018, [retrieval date 16 January 2020], internet, URL:https://www.gnpd.com/sinatra/recordpage/5387529/, item description, component | 1, 3-4 |
| A | JP 2011-503059 A (MOMENTIVE PERFORMANCE MATERIALS INC.) 27.01.2011 (2011-01-27) entire text | 1-6 |
| A | EP 3295820 A1 (THE PROCTER & GAMBLE COMPANY) 21.03.2018 (2018-03-21) entire text, all drawings | 1-6 |

☐ Further documents are listed in the continuation of Box C.  ☒ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 16 January 2020 (16.01.2020) | 04 February 2020 (04.02.2020) |

| Name and mailing address of the ISA/ Japan Patent Office 3-4-3, Kasumigaseki, Chiyoda-ku, Tokyo 100-8915, Japan | Authorized officer |
| --- | --- |
| | Telephone No. |

Form PCT/ISA/210 (second sheet) (January 2015)

| INTERNATIONAL SEARCH REPORT | | International application No. | |
|---|---|---|---|
| Information on patent family members | | PCT/JP2019/042714 | |

| Patent Documents referred in the Report | Publication Date | Patent Family | Publication Date |
|---|---|---|---|
| JP 2011-503059 A | 27 Jan. 2011 | US 2010/0310491 A1 entire text WO 2009/061360 A1 EP 2214633 A1 KR 10-2010-0084645 A CN 101909598 A | |
| EP 3295820 A1 | 21 Mar. 2018 | (Family: none) | |

Form PCT/ISA/210 (patent family annex) (January 2015)

**EP 3 875 155 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4805482 B
- JP 2016525534 A

- JP 5947340 B